# EUROPEAN PATENT SPECIFICATION

(11) **EP 1 974 716 B1**
(45) Date of publication and mention of the grant of the patent: **28.03.2012**
(21) Application number: 07105397.9
(22) Date of filing: 30.03.2007
(51) Int. Cl.: A61K 8/26, A61K 8/04, A61Q 15/00, A61Q 9/02

(54) **Cosmetic composition comprising an aluminium salt**
Kosmetische Zusammensetzung mit Aluminiumsalz
Composition cosmétique comportant du sel d'aluminium

(43) Date of publication of application: 01.10.2008
(73) Proprietor: Unilever N.V., 3013 AL Rotterdam (NL)
(72) Inventor: de Beer, Andrew, Iwan, 2719 CK Zoetermeer (NL); Campbell, Stuart, John, 2292 AH Wateringen (NL)
(74) Representative: van Loon, C.J.J.

(56) References cited:
- WO-A-2006/133725
- GB-A- 1 254 534
- JP-A- 58 057 313
- US-A- 2 211 805
- US-A- 3 111 703
- US-A- 5 348 735

## Description

The invention relates to a cosmetic composition comprising an aluminium salt. The invention further relates to a method for preparing such a composition.

Aluminium salts may be used in cosmetic applications for several purposes. For instance, aluminium chlorides (hexahydrate) are used as antiperspirants. Alum (aluminium sulphate dodecahydrates) is used to reduce body odour in deodorants or as an astringent.

The inventors have found that in applications wherein an aluminium salt is used in a particulate form, agglomeration of the particles may result in a problem with respect to applying the composition. In particular, the agglomeration has been found detrimental to the flowing properties of particles comprising an aluminium salt.

More in particular, the agglomeration of the particles may cause at least a partial blocking of the outlet or outlets from a container from which the composition may be applied. Such problem may especially be prominent in case the outlet or outlets are relatively small, such as in containers provided with a spray dispenser (such as aerosol containers or pump spray containers) or containers wherein the outlet or outlets are narrow in at least one dimension, such as in roll-on applications.

Also, the agglomeration of particles may results in the formation of agglomerates of a size which can result in a unpleasant sensation or even a detrimental effect when applied to the body, *e.g.* when applied to the skin by hand.

It is an object of the present application to provide a novel cosmetic composition comprising an aluminium salt.

In particular it is an object of the invention to provide a novel cosmetic composition comprising such particles with good flowing properties.

One or more other objects which may be solved in accordance with the invention, will be apparent from the remainder of the description and/or the claims.

It has now been found that one or more problems underlying the invention are solved by providing a cosmetic composition comprising an aluminium salt in a specific form.

Accordingly, the present invention relates to a cosmetic composition comprising an at least partially dehydrated aluminium sulphate salt and a carrier liquid other than water which carrier liquid is essentially free of water, wherein the water of hydration saturation level of the salt is less than 90 mol %

It has been found that an at least partially dehydrated aluminium sulphate salt in a cosmetic composition (also comprising a carrier liquid, which usually is essentially water-free), shows good flowing properties. Agglomeration of primary particles of the aluminium salt is generally avoided, or at least the agglomeration is such that a detrimental effect as a result of excessive agglomeration is generally avoided or at least reduced. In particular, at least a partial blocking of the outlet or outlets from a container from which the composition may be applied can be avoided or at least reduced. In an embodiment, the formation of agglomerates of a size which can result in a unpleasant sensation is avoided or at least reduced.

In particular, the salt in the composition has been found to have good free-flowing properties. The term "free-flowing" is used for a powdered material that is not sticky, and thus has no or hardly any tendency to agglomerate or to adhere to contact surfaces.

The composition may be in particular an aerosol, a gel, a paste or a liquid dispersion. When referred herein to a specific physical condition of the composition, such as gas, liquid, solid, gel, etc., the condition of the composition at 25 °C and 1.0 bar is meant, unless specified otherwise.

The cosmetic composition may in particular be selected from the group of deodorants, antiperspirants, shaving compositions, after-shaves and perfumes.

The aluminium salt can in principle be selected from any sulphate salt suitable for use in a cosmetic composition. In particular, the salt may be selected from aluminium salts that -in fully hydrated form - can be represented by the formula M⁺₂SO₄·Al³⁺₂(SO₄)₃ ·24H₂O, or - simplified - as: M⁺Al³⁺(SO₄)₂·12H₂O. Herein, M⁺ is usually selected from the group of ammonium, alkali metal ions and combinations thereof. The alkali metal ions may in particular be selected from the group of sodium ions and potassium ions. In principle, a part of the M⁺ ions may be formed by H⁺.

The aluminium salt is at least partially dehydrated, which means that the water of hydration is less than in water-hydration-saturated aluminium salt crystals, *i.e.*, in the above formulae, at least partially dehydrated means to have less than 12 water molecules per Al³⁺. Usually, the water of hydration saturation level of the salt is less than 90 mol %, in particular 87.5 mol % or less (on average up to 10 water molecules per Al³⁺ in the above formulae).

For good (free-)flowing properties it is in particular preferred that the water of hydration saturation level of the salt is up to 75 mol % (on average up to 9 water molecules per Al³⁺ in the above formulae). More in particular, the water of hydration saturation level may be up to 50 mol %(on average up to 6 water molecules per Al³⁺ in the above formulae) or up to 25 mol % (on average up to 3 water molecules per Al³⁺ in the above formulae). It is envisaged that a low level of saturation may be advantageous in prolonging the storage stability of the cosmetic composition.

For practical reasons, some residual water of hydration may be present, such as 1 mol % or more, 6.25 mol % or more (on average at least 1 water molecule per Al³⁺ in the above formulae) or 12.5 mol % or more (on average at least 2 water molecules per Al³⁺ in the above formulae).

In an embodiment the salt is anhydrous, which means in particular that the residual amount of water in the crystals of the salt is below the detection level.

The amount of water of hydration in the aluminium salt and the level of saturation can be determined by drying the aluminium salt at 200°C till constant weight, the difference in weight loss is expressed as amount of water of hydration. The level of saturation may be calculated by comparing the weight loss against a fully hydration saturated aluminium salt.

In general, most or all of the aluminium salt is present as particles. As used herein, the term "particles" relates to a solid material (the aluminium salt) that is not molecularly dissolved in carrier liquid. The maximum desired size is determined to some extent to the type of cosmetic composition and/or the type of container from which the cosmetic composition is dispensed, when used.

In particular in view of avoiding at least partial blocking of an outlet of a container containing the composition (such as an aerosol) and/or for avoiding an unpleasant sensation, *e.g.* to the skin, when a composition (such as a stick or roll-on composition), it is preferred that at least 90 vol % of the salt, in particular at least 95 vol %, more in particular at least 99 vol % of the salt is present in the form of particles having a size of less than 200 µm, more preferably of less than 100 µm, even more preferably a size of less than 50 µm. Herein, particles are considered to have a size of less than 200 µm, less than 100 µm or less than 50 µm respectively, if the particles passes through a sieve having a mesh size of 200 µm, respectively 100 µm respectively 50 µm, in particular with a sieve according to DIN ISO 3310.

The lower limit for the size is not critical for, e.g. avoiding blocking. For practical reasons, usually at least 90 vol % of the salt may have a size of at least 1 µm.

The concentration of aluminium salt particles may be chosen within a wide range, depending upon intended the purpose for the composition or the application form (*e.g.* aerosol, stick or roll-on). Usually, the concentration is at least 0.2 wt. %, in particular at least 0.5 wt. %, preferably at least 1 wt. %, in particular at least 2 wt. %, more in particular at least 4 wt. %, based on total liquid(s) and solid(s). Usually, the concentration is up to 75 wt. %, based on total liquid(s) and solid(s). For a highly homogenous distribution of the salt in the carrier liquid, the concentration usually is up to 50 wt. %, in particular up to 40 wt. %, more in particular up to 30 wt. %, based on total liquid(s) and solid(s).

The composition in general comprises a carrier liquid other than water. The carrier liquid is usually a non-aqueous liquid, and is essentially free of water. In particular, a liquid is considered essentially free of water, if the water content is too low to cause visual agglomeration of the salt particles. More in particular, a liquid is considered essentially free of water if the water content of the liquid is less than 0.5 wt. %.

One or more essentially non-polar compounds are particular suitable for forming the carrier liquid, as in such compounds the aluminium salt typically remains substantially undissolved. In principle, it is possible though to provide a composition of the invention with one or more polar liquid compounds.

In a particularly preferred embodiment, the carrier liquid comprises at least one compound selected from the group of silicone oils and liquid organic compounds. (i.e. liquid at 25 °C).

The silicone oil may be volatile or non-volatile. As used herein, "volatile" refers to those materials which have a measurable vapour pressure at ambient conditions (25 °C). Such volatile silicone oils may be cyclic or linear. A description of various volatile silicone oils is found in Todd, et al., "Volatile Silicone Fluids for Cosmetics", 91 Cosmetics and Toiletries, 27-32 (1976), incorporated by reference herein. Preferred volatile silicone oils include those having from about 3 to about 9 silicon atoms. Cyclic volatile silicones useful herein include cyclic dimethylsilicones, wherein the number of silicon atoms preferably is 3-7. Linear volatile silicone oils include those of the formula: (CH3)₃Si-O-[Si(CH₃)₂-O]n-Si(CH₃), wherein n preferably is 1 to 7. Linear volatile silicones generally have viscosities of less than about 5·10⁻⁶ m²·s⁻¹ (5 centistokes) at 25 °C, whereas the cyclic silicones generally have viscosities of less than about 10·10⁻⁶ m²·s⁻¹ (10 centistokes). Examples of volatile silicone oils useful in the present invention include: Dow Corning 344, Dow Corning 345 and Dow Corning 200 (sold by Dow Corning Corporation); 7207 and 7158 (sold by General Electric Company); and SWS-03314 (sold by SWS Silicones Corporation). In particular, good results have been achieved with cyclomethicone.

Non-volatile polyalkyl siloxanes useful herein include polydimethyl siloxanes with viscosities of from about 5 to about 100,000·10⁻⁶ m²·s⁻¹ (100,000 centistokes) at 25 °C. Such polyalkyl siloxanes include the Vicasil series (sold by General Electric Company) and the Dow Corning 200 series (sold by Dow Corning Corporation). Polyalkylaryl siloxanes include poly methylphenyl siloxanes having viscosities of from about 15 to about 65·10⁻⁶ m²·s⁻¹ (65 centistokesl at 25 °C. These are available, for example, as SF 1075 methylphenyl fluid (sold by General Electric Company) and 556 Cosmetic Grade Fluid (sold by Dow Corning Corporation). Useful polyether siloxane copolymers include, for example, a polyoxyalkylene ether copolymer having a viscosity of about 1200·10⁻⁶ to 1500·10⁻⁶ m²·s⁻¹ (1200 to 1500 centistokesl at 25 °C. Such a fluid is available as SF-1066 organosilicone surfactant (sold by General Electric Company). Polysiloxane ethylene glycol ether copolymers are preferred copolymers for use in the present compositions.

In particular one or more liquid organic compounds may be selected from the group of liquid alkanes; esters, in particular selected from the group of alkyl esters, benzoate esters, fatty acid esters and fatty alcohol esters; hydrogenated polyalkenes; glycols; and ethers, in particular PPG-ethers.

Preferred liquid organic compounds include liquid compounds selected from the group of isopropyl myristate, isopropylpalmitate, polyorganosilicones (e.g. phenyl-silicone), hexylene glycol, dipropylene glycol, ethanol, poly-lower alkoxylated cetyl alcohols, di-n-butylphthalate, diethyl sebacate, di-isopropyl adipate, neopentylglycol dihexanoate, hydrogenated polydecene, PPG-14 butyl ether, and o-ethyl, ethyl-carboxylmethyl phthalate, including mixtures thereof.

The concentration of the carrier liquid, in particular the essentially non-polar liquid, may be chosen within a wide range, depending upon the intended purpose for the composition (*e.g.* deodorant or antiperspirant) or the application form (*e.g.* aerosol, stick or roll-on). Usually, the concentration is at least 25 wt. %. For a highly homogenous distribution of the salt in the carrier liquid, the carrier liquid concentration is preferably at least 50 wt. %, more preferably at least 60 wt. %, in particular at least 70 wt. %, more in particular at least 80 wt. %, based on total liquid(s) and solid(s). Usually, the concentration is up to 99.5 wt. %, preferably up to 98 wt. %, in particular up to 96 wt. %, more in particular up to 90 wt. %, based on total liquid(s) and solid(s).

The composition may further comprise one ore more additives. The skilled person will generally be able to choose one or more suitable additives, depending upon the purpose for the composition or the application form, based on common general knowledge and the information disclosed herein.

In particular, the composition may comprise at least one additive selected from the group of fragrances; thickeners, in particular organic thickeners; gelling agents, in particular organic gelling agents; and free flowing agents, such as hectorite clay, silicagel.

In particular, an aerosol may benefit from the invention. The inventors have found that an aluminium salt that is not at least partially dehydrated very easily agglomerates in an carrier liquid for an aerosol, such that the outlet of a container comprising an aerosol comprising the salt is readily blocked by the agglomerates. Further, it is envisaged that a larger portion of the aluminium salt is actually available to be dispensed from the container, as - contrary to large agglomerates - the salt particles in a composition of the invention will generally remain small enough to be sprayed out of the container.

An aerosol according to the invention at least comprises an aluminium salt, a carrier liquid and a propellant. The propellant preferably is a gas selected from propane, isobutane, n-butane and dimetheyl ether. Other suitable propellants are known in the art, *e.g.* an inorganic gas or other gaseous organic compound.

In an aerosol of the invention the weight to weight ratio aluminium salt to carrier liquid usually is in the range of 0.5:99.5 to 50:50, in particular in the range of 10:90 to 50:50, more in particular in the range of 20:80 to 50:50. The propellant concentration in an aerosol of the invention is usually at least 50 wt. %, preferably at least 70 wt. %, in particular at least 75 wt. % based on the weight of the aerosol. The concentration is usually up to 99.5 wt. %, in particular up to 99.0 wt. %, more in particular up to 95 wt. %, based on total weight of the aerosol.

The aerosol may be present in any container suitable for holding an aerosol. Such containers are generally known in the art.

In a further advantageous embodiment, the cosmetic composition is a roll-on composition. Such a composition, usually is a liquid dispersion. Such a composition may suitably be provided in a container provided with a roller applicator.

In a further embodiment, the composition is a sprayable composition, in a container provided with a pump spray. The invention further relates to a method for preparing a composition according to the invention, comprising combining an at least partially dehydrated aluminium salt, in particular a so called burnt aluminium salt, with the carrier liquid.

Heating (burning) a hydrated aluminium salt to a temperature at which at least part of the water of hydration is removed (evaporated) from the salt crystals is a convenient way of obtaining the at least partially dehydrated salt. Depending upon the specific salt and the desired degree of dehydration (percentage of full saturation with water of hydration) a temperature and duration may be chosen. Usually the aluminium salt is heated to a temperature above the flow temperature. A temperature of at least 60 °C is usually sufficient to remove at least a portion (*e.g.* about 25 %) of the water of hydration. For a low saturation with hydration water and/or a fast dehydration time, the temperature usually is at least 90 °C, preferably at least 150 °C, at least 200 °C or at least 220 °C. For practical reasons the temperature, usually is 500 °C or less, in particular 300 °C or less, more in particular 270 °C or less, or 250 °C or less.

The heating is continued to allow sufficient water to evaporate. When heating to a temperature above the flow temperature, preferably the heating is continued until the salt solidifies.

From the at least partially dehydrated salt, particles of a desired size are formed. This may suitably be accomplished by grinding or another technique.

If desired, salt particles of a suitable size can be selected, in particular by sieving.

Combining the salt with the carrier liquid and optional other ingredients can be accomplished in a conventional manner.

The invention will now be illustrated by the following examples.

### Example 1

10 g burnt alum particles (particle size < 50 Micron) having about 7 wt. % residual water of hydration (on average less than 2 water molecules per Al³⁺) or having about 36 wt. % residual water (on average about 9 water molecules per Al³⁺) (were mixed with 60 g of cyclomethicone in a flask, sealed and stored for 4 weeks at 45°C.

The same procedure was followed using alum that had not been dehydrated (*i.e.* having 12 water molecules per Al³⁺).

After 4 weeks the mixtures were evaluated. It was found that the burnt alum particles had remained substantially unagglomerated, whereas the particles of the fully hydrated particles had form large agglomerates and caking behaviour.

### Example 2: aerosol composition

| | |
|---|---|
| Cyclomethicone | 5-10 wt.% |
| Burnt potassium aluminium sulfate | 2-5 wt. % |
| Disteardimonium hectorite | 1-3 wt. % |
| Fragrance | 0.5 - 1.5 wt. % |
| Propellant (butane,propane) qs. | |

### Example 3: roll-on composition (antideodorant)

| | |
|---|---|
| Cyclomethicone | 70 wt.% |
| Disteardimonium Hectorite | 3 wt. % |
| Propylene carbonate | 1 wt.% |
| Burnt Potassium Aluminium sulfate | 25 wt.% |
| Fragrance | 1 wt. % |

## Claims

1. Cosmetic composition comprising an at least partially dehydrated-aluminium sulphate salt and a carrier liquid, other than water, which carrier liquid is essentially free of water, wherein the water of hydration saturation level of the salt is less than 90 mol %.

2. Cosmetic composition according to claim 1, wherein the water of hydration saturation level of the salt is up to 75 mol %, in particular up to 50 mol % , more in particular up to 25 mol %.

3. Cosmetic composition according to claim 1 or 2, wherein the salt is a potassium aluminium sulphate, an ammonium aluminium sulphate or a sodium aluminium sulphate.

4. Cosmetic composition according to any of the preceding claims, wherein the carrier liquid is an non-polar liquid,
preferably a liquid comprising one or more compounds selected from the group of silicone oils and liquid hydrocarbons, which are optionally substituted.

5. Cosmetic composition according to any of the preceding claims wherein the composition is a deodorant, a shaving composition, an after-shave or a perfume.

6. Composition according to any of the preceding claims, wherein at least 90 vol % of the salt, in particular at least 95 vol %, more in particular at least 99 vol % of the salt is present in the form of particles having a size of less than 200 µm, preferably a size of less than 50 µm.

7. Composition according to any of the preceding claims, comprising at least one additive selected from the group of fragrants, thickeners, gelling agents and free flowing agents.

8. Composition according to any of the preceding claims, wherein the composition is an aerosol.

9. Composition according to claim 8, wherein the aerosol comprises at least 50 wt. % gas, preferably 70 to 99 wt. %, in particular 75 to 95 wt. %, based on total weight of the aerosol.

10. Aerosol container, comprising an aerosol according to claim 8 or 9.

11. Container provided with a roller applicator, wherein the container comprises a composition according to any of the claims 1-7.

12. Method for preparing a composition according to any of the claims 1-7, comprising combining a burnt aluminium sulphate salt with the carrier liquid.

13. Method according to claim 12, wherein the burnt aluminium sulphate salt is obtained by heating hydrated aluminium salt to a temperature above 60 °C, in particular above 150 °C, preferably in the range of 200-300 °C.

## Patentansprüche

1. Kosmetikzusammensetzung, die ein wenigstens partiell dehydratisiertes Aluminiumsulfatsalz und eine von Wasser verschiedene Trägerflüssigkeit umfasst, wobei die Trägerflüssigkeit im Wesentlichen frei von Wasser ist, wobei der Hydratwasser-Sättigungsgrad des Salzes kleiner als 90 Mol-% ist.

2. Kosmetikzusammensetzung gemäß Anspruch 1, wobei der Hydratwasser-Sättigungsgrad des Salzes bis zu 75 Mol-%, insbesondere bis zu 50 Mol-%, insbesondere bis zu 25 Mol-%, beträgt.

3. Kosmetikzusammensetzung gemäß Anspruch 1 oder 2, wobei das Salz ein Kaliumaluminiumsulfat, ein Ammoniumaluminiumsulfat oder ein Natriumaluminiumsulfat ist.

4. Kosmetikzusammensetzung gemäß einem der vorstehenden Ansprüche, wobei die Trägerflüssigkeit eine unpolare Flüssigkeit ist, vorzugsweise eine Flüssigkeit, die eine oder mehrere Verbindungen umfasst, die aus der Gruppe der Silikonöle und flüssigen Kohlenwasserstoffe, die gegebenenfalls substituiert sind, ausgewählt sind.

5. Kosmetikzusammensetzung gemäß einem der vorstehenden Ansprüche, wobei die Zusammensetzung ein Deodorant, eine Rasierzusammensetzung, ein Aftershave oder ein Parfüm ist.

6. Zusammensetzung gemäß einem der vorstehenden Ansprüche, wobei wenigstens 90 Vol.-% des Salzes, insbesondere wenigstens 95 Vol.-%, insbesondere wenigstens 99 Vol.-% des Salzes in Form von Teilchen mit einer Größe von weniger als 200 µm, vorzugsweise einer Größe von weniger als 50 µm, vorliegen.

7. Zusammensetzung gemäß einem der vorstehenden Ansprüche, die wenigstens ein Additiv umfasst, das aus der Gruppe der Duftstoffe, Verdickungsmittel, Geliermittel und Rieselhilfsmittel ausgewählt ist.

8. Zusammensetzung gemäß einem der vorstehenden Ansprüche, wobei die Zusammensetzung ein Aerosol ist.

9. Zusammensetzung gemäß Anspruch 8, wobei das Aerosol wenigstens 50 Gew.-% Gas, vorzugsweise 70 bis 99 Ges:-%, insbesondere 75 bis 95 Gew.-%, bezogen auf das Gesamtgewicht des Aerosols, umfasst.

10. Aerosolbehälter, der ein Aerosol gemäß Anspruch 8 oder 9 umfasst.

11. Behälter, der mit einem Rollapplikator versehen ist, wobei der Behälter eine Zusammensetzung gemäß einem der Ansprüche 1 bis 7 umfasst.

12. Verfahren zur Herstellung einer Zusammensetzung gemäß einem der Ansprüche 1 bis 7, das das Kombinieren eines gebrannten Aluminiumsulfatsalzes mit der Trägerflüssigkeit umfasst.

13. Verfahren gemäß Anspruch 12, wobei das gebrannte Aluminiumsulfatsalz **dadurch** erhalten wird, dass man hydratisiertes Aluminiumsalz auf eine Temperatur von über 60 °C, insbesondere über 150 °C, vorzugsweise im Bereich von 200 bis 300 °C, erhitzt.

## Revendications

1. Composition cosmétique comprenant un sel sulfate d'aluminium au moins partiellement déshydraté et un support liquide, autre que de l'eau, lequel support liquide est essentiellement exempt d'eau, et dans laquelle composition le niveau de saturation de l'eau d'hydratation du sel est inférieur à 90 % molaire.

2. Composition cosmétique selon la revendication 1, dans laquelle le niveau de saturation de l'eau d'hydratation du sel est inférieur à 75% molaire, en particulier inférieur à 50% molaire, plus particulièrement inférieur à 25% molaire.

3. Composition cosmétique selon la revendication 1 ou 2, dans laquelle le sel est un sulfate de potassium-aluminium, un sulfate d'ammonium-aluminium ou un sulfate de sodium-aluminium.

4. Composition cosmétique selon une quelconque des revendications précédentes, dans laquelle le support liquide est un liquide non polaire, de préférence un liquide comprenant un ou plusieurs composés choisis dans le groupe formé par les huiles de silicone et hydrocarbures liquides, lesquels sont éventuellement substitués.

5. Composition cosmétique selon une quelconque des revendications précédentes, dans laquelle la composition est un déodorant, une composition de rasage, une lotion après rasage ou un parfum.

6. Composition selon une quelconque des revendications précédentes, dans laquelle au moins 90 vol% du sel, en particulier au moins 95 vol%, plus particulièrement au moins 99 vol% du sel est présent sous forme de particules ayant une dimension inférieure à 200 µm, de préférence une dimension inférieure à 50 µm.

7. Composition selon une quelconque des revendications précédentes, comprenant au moins un additif choisi dans le groupe des produits odorants, agents épaississants, agents gélifiants et agents anti-massants.

8. Composition selon une quelconque des revendications précédentes, dans laquelle la composition est un aérosol.

9. Composition selon la revendication 8, dans laquelle l'aérosol comprend au moins 50% en poids de gaz, de préférence 70 à 99% en poids, en particulier 75 à 95% en poids, exprimés par rapport au poids total de l'aérosol.

10. Conteneur d'aérosol comprenant un aérosol selon la revendication 8 ou 9.

11. Conteneur pourvu d'un rouleau d'application, dans lequel le conteneur comprend une composition selon une quelconque des revendications 1 à 7.

12. Procédé de préparation d'une composition selon une quelconque des revendications 1 à 7, comprenant la combinaison d'un sel d'aluminium brûlé avec un support liquide.

13. Procédé selon la revendication 12, dans lequel le sel sulfate d'aluminium brûlé est obtenu par chauffage de sel d'aluminium hydraté à une température supérieure à 60°C, en particulier supérieure à 150 °C, de préférence dans la gamme de 200 à 300°C.
